# EUROPEAN PATENT SPECIFICATION

(11) **EP 2 198 776 B1**
(45) Date of publication and mention of the grant of the patent: **01.08.2018**
(21) Application number: 09179472.7
(22) Date of filing: 16.12.2009
(51) Int. Cl.: A61B 5/0205, A61B 5/0215, A61B 5/08

(54) **Respiratory function measuring apparatus**
Atemfunktionsmessvorrichtung
Appareil de mesure de la fonction respiratoire

(30) Priority: 22.12.2008 JP 2008326242
(43) Date of publication of application: 23.06.2010
(73) Proprietor: Nihon Kohden Corporation, Shinjuku-ku Tokyo (JP)
(72) Inventor: Ukawa, Teiji, Shinjuku-ku Tokyo (JP)
(74) Representative: Grünecker Patent- und Rechtsanwälte PartG mbB

(56) References cited:
- US-A1- 2004 040 560
- US-A1- 2004 249 297
- US-A1- 2007 000 494
- US-A1- 2007 073 170
- US-A1- 2008 033 306
- US-A1- 2008 243 016

## Description

### BACKGROUND OF THE INVENTION

The present invention relates to a respiratory function measuring apparatus which can measure a respiratory function signal indicative of an intrathoracic pressure change and the like from the invasive blood pressure.

In a recent respiration control, the ventilation mode in which spontaneous respiration remains is sometimes used, and the measurement of a work of breathing and the like attracts attention. In the measurement of a work of breathing, the measurement of the intrathoracic pressure is necessary, but it is impossible to measure the intrathoracic pressure. Therefore, the measurement of the esophageal pressure is used as a substitute for that of the intrathoracic pressure. However, the measurement of the esophageal pressure requires a cumbersome work of inserting a balloon catheter into the esophagus, and moreover has a problem in that the burden of the subject is large.

On the other hand, the central venous pressure (CVP) is considered as an important parameter in the circulatory control such as blood transfusion, and, in a severe case such as that where artificial respiration is performed, the central venous pressure is frequently measured. The heart is in the intrathoracic cavity, and hence affected by the intrathoracic pressure. In the vicinity of the right atrium, particularly, the central venous pressure is low, and hence strongly reflects the intrathoracic pressure. This relationship is acknowledged (see 0018 to 0021 of Japanese Patent No. 3,857,684).

However, the display of a blood pressure waveform is intended to faithfully display waveform information obtained from a pressure transducer, and hence has frequency characteristics of about 0 to 10 Hz or 20 Hz. Therefore, it is difficult to read respiratory variation. On the other hand, the compliance of a blood vessel has been measured from the mean blood pressure, the CVP, or the like (see U.S. Patent No. 6,315,735). However, a technique for easily monitoring an intrathoracic pressure change including information useful for the respiration control has not yet been developed.

A related-art technique in which a plurality of harmonic components are separated from a pressure waveform by using the heart rate or the breathing rate as a basic frequency, thus the respiratory effect is removed, has been proposed (for example, JP-A-2008-36433). However, the related-art technique relates to measurement of an influence which is exerted on the circulatory function by an intrathoracic pressure change due to respiration, and obtains a ratio of the cardiac frequency component of the blood pressure derived from the heart and that derived from respiration, from a frequency power spectrum obtained by the Fourier transform. In the related-art technique, the process is complicated, and waveform analysis on the time axis is required in the measurement of the respiratory function, so that analysis cannot be performed on the frequency axis of the frequency power spectrum. Moreover, a predetermined number of data must be collected in order to perform the Fourier transform. When real time processing of a signal is considered, there is a concern that a time delay is caused by the collection. In the case where the Fourier transform is performed in a state of an insufficient number of collected data, there is a possibility that the accuracy of an output signal is low.

### SUMMARY

It is therefore an object of the invention to provide a respiratory function measuring apparatus in which components derived from cardiac contraction can be removed from a central venous pressure waveform that is measured for the purpose of the circulatory control, and respiratory variation of the intrathoracic pressure can be easily estimated.

In order to achieve the object, according to the invention as defined by the independent claim, there is provided a respiratory function measuring apparatus comprising:
a first sensor configured to detect an invasive blood pressure;
a second sensor configured to measure frequency of at least one of a heart beat and a respiration; and
a controller configured to extract a respiratory function signal from the invasive blood pressure detected by the first sensor, by using at least one of the frequency measured by the second sensor and a harmonic of the frequency.

The controller may presume the extracted respiratory function signal as an intrathoracic pressure.
when the second sensor measures the frequency of the heart beat, the controller may selectively remove components corresponding to the frequency of the heart beat and a harmonic of the frequency from a signal corresponding to the invasive blood pressure.

When the second sensor measures the frequency of the heart beat, the controller may extract a component corresponding to a frequency which is lower than the frequency of the heart beat from a signal corresponding to the invasive blood pressure.

When the second sensor measures the frequency of the respiration, the controller may extract components corresponding to the frequency of the respiration and a harmonic of the frequency from a signal corresponding to the invasive blood pressure.

The frequency of the heart beat may be measured from an electrocardiogram, a plethysmogram, or an arterial blood pressure.

The frequency of the respiration may be measured from a capnometry, a respiratory flow, an airway pressure, a transthoracic electrical impedance, or a respiratory temperature.

The invasive blood pressure may be a central venous pressure (CVP) or a peripheral venous pressure.

The respiratory function measuring apparatus may further include: a respiration variation detector. When the second sensor measures the frequency of the respiration, the second sensor may presume an end-tidal. The respiration variation detector may be configured to obtain a degree of a respiratory signal with respect to the end-tidal.

The end-tidal may be obtained from a flat portion of the invasive blood pressure, a capnometry, a respiratory flow, an airway pressure, a transthoracic electrical impedance, or a respiratory temperature.

The respiratory function measuring apparatus may further include: a respiration determiner configured to, based on a polarity of a degree of the respiratory signal, determine whether the respiration is spontaneous respiration or artificial respiration.

The respiratory function measuring apparatus may further include: a secondary respiratory function calculator configured to, based on a level of the respiratory signal, obtain at least one of a PTP, an intrinsic PEEP, a work of breathing, and a P0.1.

The respiratory signal may be displayed as a painted-out portion based on a timing of the end-tidal.

A mark indicative of a timing of the end-tidal may be displayed with a waveform.

A waveform display may be performed with plotting the respiratory signal as an abscissa, and a respiratory volume which is obtained by integrating a respiratory flow, as an ordinate.

### BRIEF DESCRIPTION OF THE DRAWINGS

Fig. 1 is a diagram showing an equivalent circuit of a respiratory system.
Fig. 2 is a block diagram showing the configuration of an embodiment of the respiratory function measuring apparatus of the invention.
Fig. 3 is a block diagram showing the configuration of main portions of the embodiment of the respiratory function measuring apparatus of the invention.
Fig. 4 is a flowchart showing a process extracting a respiratory function signal in the embodiment of the respiratory function measuring apparatus of the invention.
Fig. 5 is a flowchart showing the process extracting the respiratory function signal in the embodiment of the respiratory function measuring apparatus of the invention.
Figs. 6A and 6B are views showing an example of a respiratory function signal which is displayed in the embodiment of the respiratory function measuring apparatus of the invention.
Figs. 7A and 7B are views showing an example of a respiratory function signal which is displayed in the embodiment of the respiratory function measuring apparatus of the invention.
Fig. 8 is a waveform chart illustrating a process of measuring the degree ΔCVPr of a respiratory component CVPr in the embodiment of the respiratory function measuring apparatus of the invention.
Fig. 9 is a waveform chart illustrating a process of measuring the degree ΔCVPr of the respiratory component CVPr in the embodiment of the respiratory function measuring apparatus of the invention.
Fig. 10A is a view showing a display example in which a respiratory parameter and a circulatory parameter are displayed in the same time phase in the embodiment of the respiratory function measuring apparatus of the invention.
Fig. 10B is a view showing a display example in which a respiratory parameter and a circulatory parameter are displayed in the same time phase in the embodiment of the respiratory function measuring apparatus of the invention.
Fig. 10C is a view showing a display example in which a respiratory parameter and a circulatory parameter are displayed in the same time phase in the embodiment of the respiratory function measuring apparatus of the invention.
Fig. 10D is a view showing a display example in which a respiratory parameter and a circulatory parameter are displayed in the same time phase in the embodiment of the respiratory function measuring apparatus of the invention.

### DETAILED DESCRIPTION OF EMBODIMENTS

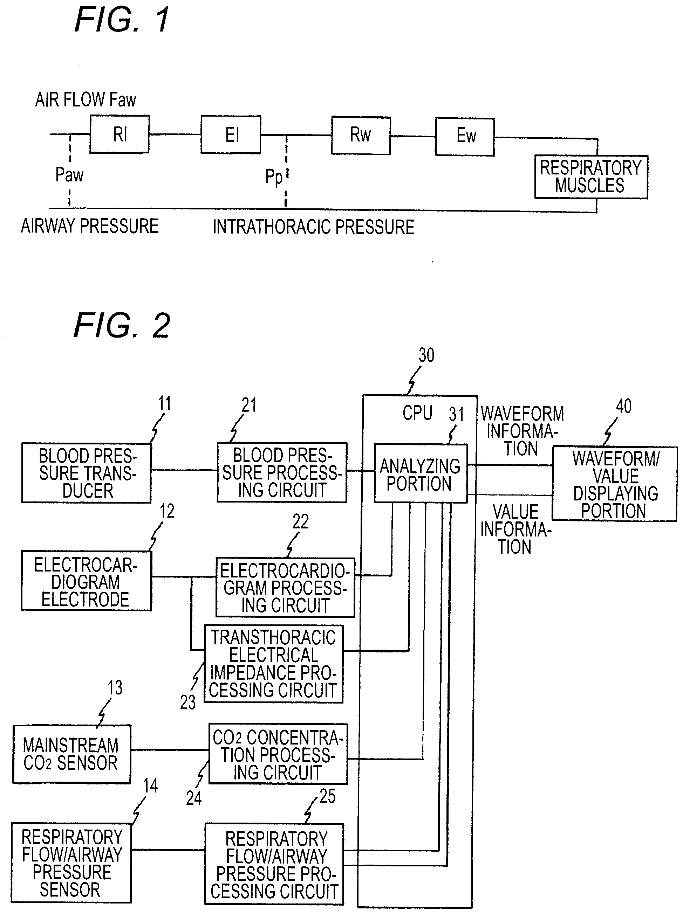

Hereinafter, an embodiment of the respiratory function measuring apparatus of the invention will be described with reference to the accompanying drawings. In the figures, the identical components are denoted by the same reference numerals, and duplicated description will be omitted. First, the principle of respiration will be described. An equivalent circuit of a respiratory system is as shown in Fig. 1. Respiration is caused by the respiratory muscles mainly configured by the diaphragm. In order to cause the lung and the thoracic to operate, a work on the lung elastance E1 and the thoracic wall elastance Ew is necessary. The respiratory flow is blocked by the airway resistance R1 and the thoracic wall resistance Rw. The work which is required in respiration is the sum of works on the airway resistance R1, the lung elastance E1, the thoracic wall resistance Rw, and the thoracic wall elastance Ew.

An air flow Faw is produced by the differential pressure between an airway pressure Paw and a thoracic pressure Pp1. In artificial respiration, the airway pressure Paw is set as a positive pressure, thereby feeding the air flow Faw into the lung. At this time, also the thoracic pressure Pp1 is a positive pressure. By contrast, in the case of spontaneous respiration, the thoracic is widened by the respiratory muscles, and the thoracic pressure Pp1 is a negative pressure.

Therefore, respiration by artificial respiration, and that by the respiratory muscles can be distinguished from each other by the thoracic pressure Pp1, and hence it is possible to estimate the behavior of the respiratory muscles.

The respiratory function measuring apparatus of the embodiment of the invention employs the configuration shown in Fig. 2, or namely includes a blood pressure transducer 11, electrocardiogram electrodes 12, a mainstream CO₂ sensor 13, and a respiratory flow/airway pressure sensor 14.

A blood pressure processing circuit 21 is connected to the blood pressure transducer 11 which is a blood pressure sensor for detecting the invasive blood pressure. An output signal of the blood pressure processing circuit 21 is sent to a CPU 30. An electrocardiogram processing circuit 22 and a transthoracic electrical impedance processing circuit 23 are connected to the electrocardiogram electrodes 12 which constitute a sensor for measuring the frequency of the heart beat. Output signals of the electrocardiogram processing circuit 22 and the transthoracic electrical impedance processing circuit 23 are sent to the CPU 30. As a sensor for measuring the frequency of the heart beat, in place of the above-described electrocardiogram sensor, a plethysmogram sensor, an arterial blood pressure sensor, or the like may be used.

A CO₂ concentration processing circuit 24 is connected to the mainstream CO₂ sensor 13 which is a sensor for measuring the frequency of respiration. An output signal of the CO₂ concentration processing circuit 24 is sent to the CPU 30. A respiratory flow/airway pressure processing circuit 25 is connected to the respiratory flow/airway pressure sensor 14 which is a sensor for measuring the frequency of respiration. An output signal of the respiratory flow/airway pressure processing circuit 25 is sent to the CPU 30. As a sensor for measuring the frequency of respiration, a respiratory temperature sensor may be employed.

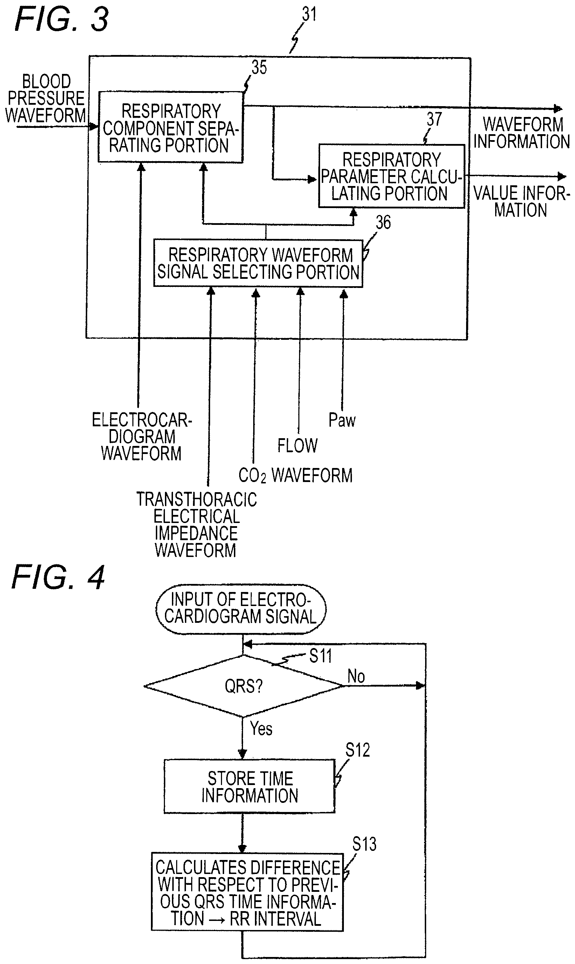

The CPU 30 includes an analyzing portion 31. All of a blood pressure waveform signal produced by the blood pressure processing circuit 21, an electrocardiogram waveform signal produced by the electrocardiogram processing circuit 22, a transthoracic electrical impedance waveform signal produced by the transthoracic electrical impedance processing circuit 23, a CO₂ concentration waveform signal produced by the CO₂ concentration processing circuit 24, and a respiratory flow signal (FLOW in Fig. 3) and airway pressure Paw signal (Paw in Fig. 3) produced by the respiratory flow/airway pressure processing circuit 25 are taken up by the analyzing portion 31.

The analyzing portion 31 obtains the respiratory function by using the signals, and sends the respiratory function waveform and the respiratory function value to a waveform/value displaying portion 40 configured by a display such as an LCD and a display controller, to display the waveform and value related to the respiratory function.

As shown in Fig. 3, the analyzing portion 31 includes a respiratory component separating portion 35, a respiratory waveform signal selecting portion 36, and a respiratory parameter calculating portion 37. The transthoracic electrical impedance waveform signal produced by the transthoracic electrical impedance processing circuit 23, the CO₂ concentration waveform signal produced by the CO₂ concentration processing circuit 24, and the respiratory flow signal and airway pressure Paw signal produced by the respiratory flow/airway pressure processing circuit 25 are given to the respiratory waveform signal selecting portion 36. The respiratory waveform signal selecting portion 36 selects a required signal from the above-mentioned signals, and gives the selected signal to the respiratory component separating portion 35 and the respiratory parameter calculating portion 37. The criterion for the signal selection will be described later.

The blood pressure waveform signal produced by the blood pressure processing circuit 21, and the electrocardiogram waveform signal produced by the electrocardiogram processing circuit 22 are given to the respiratory component separating portion 35. Moreover, the signal selected by the respiratory waveform signal selecting portion 36 is given to the respiratory component separating portion. The respiratory component separating portion 35 extracts a respiratory function signal by using a signal derived from cardiac contraction with respect to the blood pressure waveform signal produced by the blood pressure processing circuit 21, and, in this example, outputs respiratory function waveform information.

The respiratory parameter calculating portion 37 receives the signal selected by the respiratory waveform signal selecting portion 36, and the respiratory function waveform information output from the respiratory component separating portion 35. The respiratory parameter calculating portion 37 calculates secondary respiratory functions such as a PTP (Pressure-Time Product), an intrinsic PEEP, a work of breathing, the value (P0.1) after an elapse of 0.1 seconds from the start of the respiratory effort, as respiratory parameters and by using a related-art process, and outputs these measurement results. These parameters are displayed on the waveform/value displaying portion 40.

The PTP is an index which is obtained by time integrating the intrathoracic pressure in the inspiration of spontaneous respiration, indicates the consumption of oxygen in the respiratory effort and the respiratory muscles, and is used in the determination of whether, after weaning from an artificial respirator, the patient is forced to excessively exert the respiratory effort or not. The intrinsic PEEP is an index which is obtained from an absolute value change of the intrathoracic pressure between the timing of starting the respiratory effort and that of starting the respiratory flow. The work of breathing is a work which is necessary for changing the respiratory volume against the resistances of the airway, the lung, and the thoracic. P0.1 is the value after an elapse of 0.1 seconds from the timing when the airway is instantaneously closed and the respiratory effort is started, and an evaluation index for the respiratory center function, i.e., the respiratory drive.

In the related-art technique, usually, the secondary respiratory functions are calculated by using the esophageal pressure (for example, ISHIKAWA Kiyoshi and KATSUYA Hirotada "Respiratory Function Monitor Under Mechanical Ventilation", February 1993, SHUTYU CHIRYO (vol. 2, no. 2)). When the respiratory signal which reflects the intrathoracic pressure, and which is extracted by the invention is used in place of the esophageal pressure, however, the secondary respiratory functions can be calculated.

The thus configured respiratory function measuring apparatus measures the respiratory function signal by using the central venous pressure. In this case, the blood pressure transducer 11 is set so that the central venous pressure of the subject is taken out, and the electrocardiogram electrodes 12, the mainstream CO₂ sensor 13, and the respiratory flow/airway pressure sensor 14 are attached to required positions of the subject, respectively, and then the measurement is started.

The blood pressure processing circuit 21 receives the blood pressure signal which is detected by the blood pressure transducer 11. Based on the blood pressure signal, the blood pressure processing circuit 21 calculates the blood pressure value (central venous pressure), and outputs the value as the digitized blood pressure waveform signal.

The electrocardiogram processing circuit 22 receives an electrocardiogram signal which is detected by the electrocardiogram electrodes 12. Based on the electrocardiogram signal, the electrocardiogram processing circuit 22 obtains an electrocardiogram waveform, and outputs the waveform as the digitized electrocardiogram waveform signal.

The analyzing portion 31 of the CPU 30 fetches the blood pressure waveform signal and the electrocardiogram waveform signal, and the respiratory component which is the respiratory function signal is extracted in the respiratory component separating portion 35. It is assumed that the blood pressure waveform signal is a CVP and the respiratory component to be extracted is a CVPr. The respiratory component separating portion 35 performs extraction by using a filter which allows the respiratory component CVPr to pass therethrough. The filter can be realized by a filter which removes frequency components derived from cardiac contraction, from the CVP waveform, or by a filter which selectively takes out a frequency derived from respiration, from the CVP waveform.

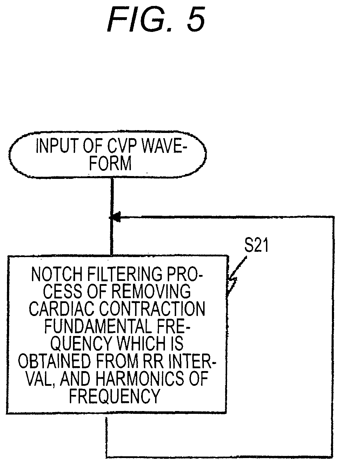

Here, a technique in which the filter is realized by a filter which removes frequency components derived from cardiac contraction, from the CVP waveform will be described. The respiratory component separating portion 35 performs the operations indicated in the flowcharts shown in Figs. 4 and 5, to conduct the filter removal of frequency components derived from cardiac contraction. Namely, the portion fetches the electrocardiogram waveform signal, and detects the incoming of a QRS wave by means of, for example, steep rising and falling of the signal value (S11).

If the incoming of a QRS wave is detected, information of the time of the detection is stored (S12), and calculates the difference with respect to the previous QRS detection time to obtain the RR interval (S13) . Thereafter, steps S11 to S13 are repeated while fetching next data.

Concurrently with the process shown in the flowchart of Fig. 4, the respiratory component separating portion 35 fetches the CVP waveform which is the blood pressure waveform signal, and performs a notch filtering process of removing a cardiac contraction fundamental frequency which is obtained from the RR interval calculated in step S13 of Fig. 4, and harmonics of the frequency, thereby extracting the respiratory function signal (hereinafter, referred to as the respiratory component CVPr) (S21).

In the configuration of the respiratory component separating portion 35, in order to separate the CVP waveform into the component derived from cardiac contraction and the respiratory component CVPr which is the component derived from respiration, a low-pass filter which allows only a frequency that is lower than the frequency of the heart beat to pass therethrough may be used. A low-pass filter which is used in the configuration can be realized in a relatively easy manner, and has an advantage that the respiratory function can be measured without relying on the performance of the CPU.

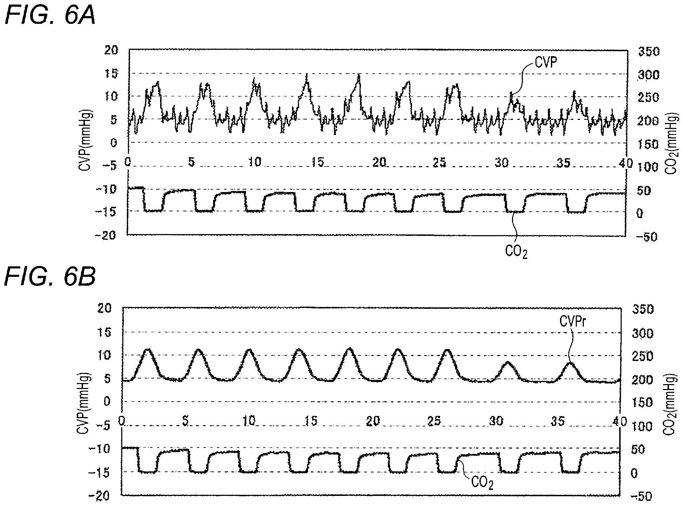

The waveform information of the respiratory component CVPr which is obtained in the above is sent to the waveform/value displaying portion 40 together with CVP waveform information and CO₂ concentration waveform information which is produced from the CO₂ concentration waveform signal sent through the respiratory waveform signal selecting portion 36. In the waveform/value displaying portion 40, as shown in Figs. 6A and 6B, for example, the information is displayed while the abscissa shows the time and the ordinate shows the volume. In Fig. 6A, the CVP waveform (the upper side in the figure) and CO₂ concentration waveform (the lower side in the figure) which are obtained from the subject to whom artificial respiration is being applied are juxtaposed. In Fig. 6B, the respiratory component CVPr waveform (the upper side in the figure) obtained by extraction from the CVP of the subject to whom artificial respiration is being applied and CO₂ concentration waveform (the lower side in the figure) are juxtaposed.

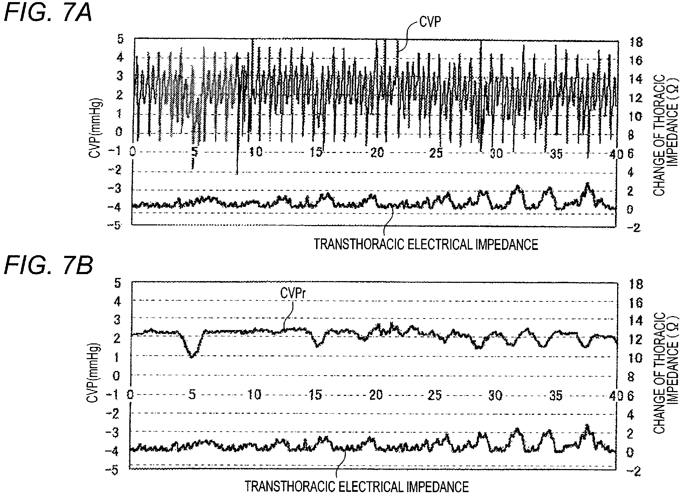

In the above, the CO₂ concentration waveform is juxtapositionally shown. Alternatively, at least one of the respiratory flow waveform, the airway pressure waveform, and the transthoracic electrical impedance waveform may be juxtapositionally shown. In the alternative, in accordance with an instruction input which is externally performed by the operator, or a previous setting, the respiratory waveform signal selecting portion 36 selects a required signal, and supplies the selected signal to the respiratory component separating portion 35 and the respiratory parameter calculating portion 37. In Fig. 7A, the CVP waveform (the upper side in the figure) and transthoracic electrical impedance waveform (the lower side in the figure) which are obtained from the subject who is performing spontaneous respiration are juxtaposed. In Fig. 7B, the respiratory component CVPr waveform (the upper side in the figure) obtained by extraction from the CVP of the subject who is performing spontaneous respiration and transthoracic electrical impedance waveform (the lower side in the figure) are juxtaposed.

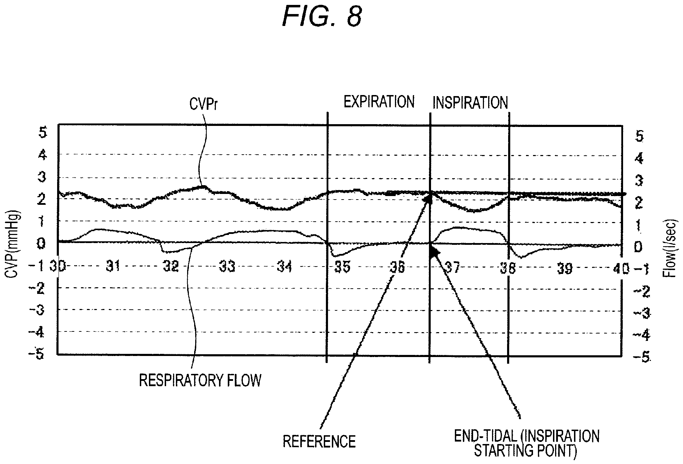

In the embodiment, the respiratory parameter calculating portion 37 calculates the degree ΔCVPr of the respiratory component CVPr with respect to the end-tidal (inspiration starting point), in a similar manner as the case of the esophageal pressure. In the case where the respiratory flow signal is obtained, the respiratory flow is changed as shown in Fig. 8, and the end-tidal (inspiration starting point) can be easily detected. Therefore, also the degree ΔCVPr of the respiratory component CVPr with respect to the end-tidal (inspiration starting point) can be easily measured. The measured ΔCVPr is sent to the waveform/value displaying portion 40, and displayed on the waveform/value displaying portion 40 together with the other respiratory parameters. On the basis of the polarity of the measured ΔCVPr, the respiratory parameter calculating portion 37 detects whether artificial respiration or spontaneous respiration is performed, and displays the result of the detection on the waveform/value displaying portion 40.

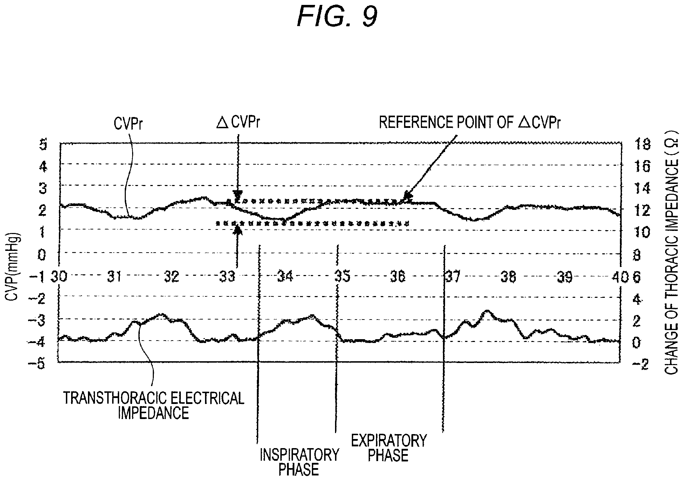

In the case where the respiratory flow signal is not obtained because of extubating a tracheal tube, the respiratory parameter calculating portion 37 determines an expiratory phase or an inspiratory phase by using the transthoracic electrical impedance. This will be described with reference to Fig. 9. In a zone which is slightly longer than one respiration period, two points of the minimum impedance which exist before and after the apex of a peak that is raised by 2.0 Ω or more from the minimum impedance are obtained. A peak portion interposed between the two points is the inspiratory phase, and a somewhat flat portion interposed between the two points is the expiratory phase.

The respiratory parameter calculating portion 37 sets the position of the respiratory component CVPr having the highest value in the expiratory phase which is obtained as described above, as the reference point of ΔCVPr. The degree of the respiratory component CVPr with respect to the reference point is measured as ΔCVPr. As described above, the measurement result is displayed on the waveform/value displaying portion 40 together with the other respiratory parameters. Also, ΔCVPr may be displayed as a respiratory parameter labeled "intrathoracic pressure change".

In the above, the end-tidal is detected from the respiratory flow or the transthoracic electrical impedance. Alternatively, a technique in which the end-tidal is detected from the flat portion of the CVP, that in which the end-tidal is detected from the CO₂ concentration waveform, that in which the end-tidal is detected from the airway pressure, or that in which the respiratory temperature is obtained and the end-tidal is detected from the temperature may be employed.

In the embodiment, the central venous pressure of the subject is taken out. Alternatively, while the blood pressure transducer 11 is used as a peripheral venous pressure sensor, the peripheral venous pressure may be taken out, and the pressure may be used. The reason that the peripheral venous pressure can be used is as follows. Since a blood vessel is filled with blood, a blood vessel can be deemed as a pressure transmission system. However, there is a flow of blood, and hence a pressure difference is produced by the vascular resistance. In the case where the peripheral venous pressure is used, the pressure difference appears as a diremption from the central venous pressure.

However, a blood flow flowing through a venous vessel can be considered as a steady flow and has substantially no arterial flow component. Therefore, the diremption appearing in this case merely appears as a DC-like offset. The object of the invention is to presume intrathoracic pressure variation from respiratory variation of the blood pressure. Therefore, a DC-like offset produced in the pressure does not cause a serious problem.

It is clinically important to know the relationship between the intrathoracic pressure and the other respiratory parameters such as the airway pressure and the thoracic motion. Therefore, it is useful to display the respiratory component waveform of the blood pressure which is obtained in the invention, as a waveform reflecting the intrathoracic pressure in the same time phase as the other respiratory parameters. However, respiratory parameters are sometimes displayed on a screen at a low sweep speed which is different from that for circulatory parameters such as an electrocardiogram. In such a case, in a related-art biological information monitor, it is impossible to observe respiratory variation of the blood pressure in the same time phase as respiratory parameters.

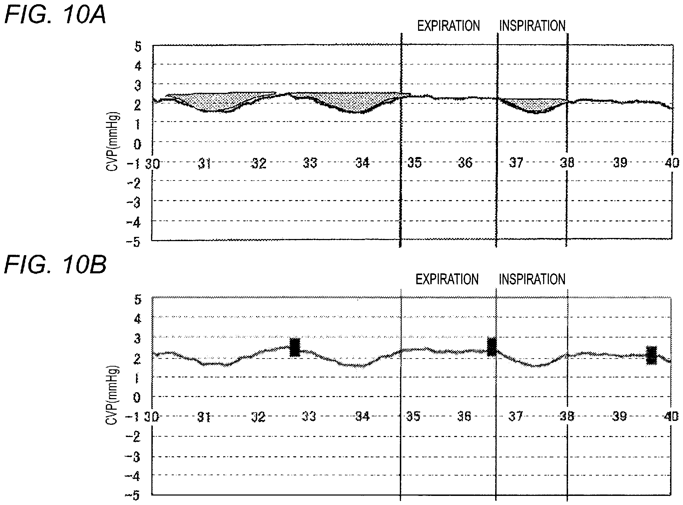

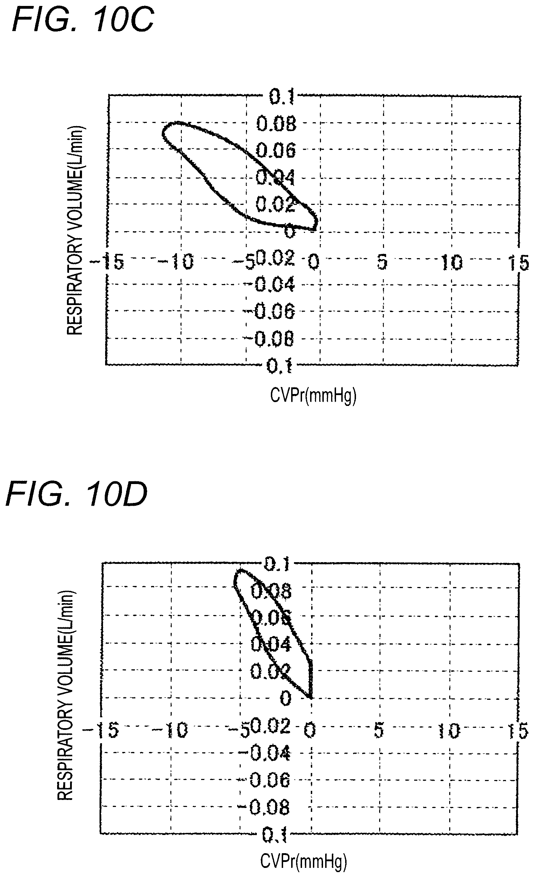

In the invention, the configuration is employed where, even when a respiratory parameter is displayed at a sweep speed which is different from that of a circulatory parameter, the respiratory parameter can be displayed in the same time phase as the circulatory parameter. Here, the respiratory parameter includes the respiratory flow, the airway pressure, the respiratory component CVPr, and the like, and the circulatory parameter includes an electrocardiogram, the arterial blood pressure, etc. As a technique for displaying a respiratory parameter and a circulatory parameter in the same time phase, the followings may be contemplated. In a first technique, the respiratory component waveform is displayed as a painted-out portion with respect to the pressure value at the timing of the end-tidal (Fig. 10A). The painted-out portions correspond to the PTP, and the respiratory effort of the patient can be known intuitively. Alternatively, another technique in which a mark indicative of the timing of the end-tidal is displayed simultaneously with the waveform display (Fig. 10B) may be employed. According to the technique, even when the measurement waveform is disturbed by a procedure such as a change in body change, it is possible to easily know whether the end-tidal detecting process normally operates or not. Furthermore, a technique (Figs. 10C and 10D) in which a waveform is displayed with plotting the CVPr as the abscissa, and the respiratory volume as the ordinate may be possible. The slope of the waveform of Fig. 10D is steeper than that of the waveform of Fig. 10C. This shows that a larger respiratory volume is obtained by a smaller respiratory effort of the patient. In spontaneous respiration, therefore, the expansibility (compliance) of the lung can be easily checked from the displayed waveform shape. In such display techniques, the respiratory function can be known more easily.

According to an aspect of the invention, the respiratory function signal is extracted from the blood pressure detected by the blood pressure sensor for detecting the invasive blood pressure, by using the frequency derived from cardiac contraction or that derived from respiration. Therefore, the apparatus has an effect that, without imposing a large burden on the subject, a respiratory function signal indicative of an intrathoracic pressure change and the like can be easily measured.

According to an aspect of the invention, the frequency of the heart beat and the harmonic component of the frequency are selectively removed, and hence it is possible to measure the respiratory function signal which is indicative of an intrathoracic pressure change and the like, and from which an influence caused by the heart beat contained in the blood pressure waveform is eliminated. When the harmonic component of the frequency of the heart beat is selectively removed by using a notch filter, particularly, a signal indicative of an intrathoracic pressure change derived from respiration can be extracted without loss of necessary information.

According to an aspect of the invention, a frequency which is lower than the measured frequency of the heart beat is allowed to pass, and hence it is possible to measure the respiratory function such as an intrathoracic pressure change from which an influence caused by the heart beat contained in the blood pressure waveform is eliminated. This is caused by the following reason. Usually, the frequency of the respiration is lower than the frequency of the heart beat. When only a frequency which is lower than the frequency of the heart beat is allowed to pass, therefore, the fundamental wave of an intrathoracic pressure change derived from respiration can be taken out. In a use in which the transition of the degree of the intrathoracic pressure change is observed, even only the fundamental wave component having no harmonic component functions as useful information. Particularly, a filter through which the low frequency band is allowed to pass can be realized relatively easily, and the respiratory function can be measured without relying on the performance of a CPU.

According to an aspect of the invention, the measured frequency of the respiration and the harmonic component of the frequency are allowed to pass, and hence only a respiration variation component can be extracted from the blood pressure waveform, so that the respiratory function such as an intrathoracic pressure change can be measured. While removing disturbance factors other than the heart beat, therefore, an intrathoracic pressure change derived from respiration can be faithfully taken out from a blood pressure signal together with the harmonic component.

According to an aspect of the invention, it is possible to determine whether respiration is spontaneous respiration or artificial respiration, and hence it is possible to obtain an index for knowing a timing of weaning from artificial respiration. In measurement of the lung compliance of the patient from the airway pressure or a respiratory flow signal in artificial respiration, for example, it is sometimes presumed that the muscles of the thoracic relax. In this case, in accordance with the timing, spontaneous respiration is removed from the measurement, whereby more accurate compliance measurement is enabled.

According to an aspect of the invention, the secondary respiratory function can be calculated, and hence indexes such as the respiratory effort of the patient and the consumption of oxygen of the respiratory muscles can be obtained. In the prior art, these indexes are measured by measuring the esophageal pressure. According to the invention, without measuring the esophageal pressure, these indexes can be estimated by using the central venous pressure which is measured often. Therefore, the management of weaning from artificial respiration can be realized effectively and easily.

According to an aspect of the invention, even when a respiratory parameter is displayed at a sweep speed which is different from that of a circulatory parameter, the blood pressure waveform can be displayed in the same time phase as the respiratory parameter. Therefore, the respiratory function can be known more easily.

According to an aspect of the invention, the respiratory signal can be displayed as a painted-out portion, and hence a PTP which will be described later can be visually emphasized by means of the area of the painted-out portion. Moreover, the respiratory effort of the patient can be known intuitively and rapidly.

According to an aspect of the invention, the mark indicative of the timing of the end-tidal is displayed simultaneously with the waveform display. Even when the measurement waveform is disturbed by a procedure such as a change in body change, therefore, the mark indicative of the end-tidal can be displayed superimposedly on the waveform, and hence it is possible to know whether the end-tidal detecting process normally operates or not.

According to an aspect of the invention, a waveform can be displayed with plotting the pressure as the abscissa, and the respiratory volume as the ordinate. Also in spontaneous respiration, the expansibility (compliance) of the lung can be easily checked from the displayed waveform shape.

## Claims

1. A respiratory function measuring apparatus comprising:
a first sensor (11) configured to detect an invasive blood pressure;
a second sensor (12, 13, 14) configured to measure frequency of at least one of a heart beat and a respiration; and
a controller (30) configured to extract a respiratory function signal from the invasive blood pressure detected by the first sensor (11), by using at least one of the frequency measured by the second sensor (12, 13, 14) and a harmonic of the frequency;
wherein the controller is configured to estimate respiratory variations of an intrathoracic pressure using the extracted respiratory function signal,
**characterized in that** estimating the respiratory variations comprises detecting a position of the extracted respiratory function signal having a highest value in an expiratory phase or detecting an end-tidal, setting that highest value or the end-tidal, respectively, as a reference point, measuring the change of the extracted respiratory function signal with respect to the reference point and setting the measured change as the respiratory variation of the intrathoracic pressure; and
wherein the respiratory function measuring apparatus is configured to display the extracted respiratory function signal and the estimated respiratory variations of the intrathoracic pressure.

2. The respiratory function measuring apparatus according to claim 1, wherein
when the second sensor is configured to measure the frequency of the heart beat, the controller is configured to selectively remove components corresponding to the frequency of the heart beat and a harmonic of the frequency from a signal corresponding to the invasive blood pressure.

3. The respiratory function measuring apparatus according to claim 1, wherein
when the second sensor is configured to measure the frequency of the heart beat, the controller is configured to extract a component corresponding to a frequency which is lower than the frequency of the heart beat from a signal corresponding to the invasive blood pressure.

4. The respiratory function measuring apparatus according to claim 1, wherein
when the second sensor is configured to measure the frequency of the respiration, the controller is configured to extract components corresponding to the frequency of the respiration and a harmonic of the frequency from a signal corresponding to the invasive blood pressure.

5. The respiratory function measuring apparatus according to claim 1, wherein
the frequency of the heart beat is measured from an electrocardiogram, a plethysmogram, or an arterial blood pressure.

6. The respiratory function measuring apparatus according to claim 1, wherein
the frequency of the respiration is measured from a capnometry, a respiratory flow, an airway pressure, a transthoracic electrical impedance, or a respiratory temperature.

7. The respiratory function measuring apparatus according to claim 1, wherein
the invasive blood pressure is a central venous pressure (CVP) or a peripheral venous pressure.

8. The respiratory function measuring apparatus according to claim 1, further comprising:
a respiration variation detector, wherein
when the second sensor is configured to measure the frequency of the respiration, the second sensor is configured to detect the end-tidal, and
the respiration variation detector is configured to measure a change of the respiratory function signal with respect to the end-tidal.

9. The respiratory function measuring apparatus according to claim 8, wherein
the end-tidal is obtained from a flat portion of the invasive blood pressure, a capnometry, a respiratory flow, an airway pressure, a transthoracic electrical impedance, or a respiratory temperature.

10. The respiratory function measuring apparatus according to claim 8, further comprising:
a respiration determiner configured to, based on a polarity of a degree of the respiratory function signal, determine whether the respiration is spontaneous respiration or artificial respiration.

11. The respiratory function measuring apparatus according to claim 8, further comprising:
a secondary respiratory function calculator configured to, based on a level of the respiratory function signal, obtain at least one of a PTP, an intrinsic PEEP, a work of breathing, and a P0.1.

12. The respiratory function measuring apparatus according to claim 8, wherein
the respiratory function signal is displayed as a painted-out portion based on a timing of the end-tidal.

13. The respiratory function measuring apparatus according to claim 8, wherein
a mark indicative of a timing of the end-tidal is displayed with a waveform.

14. The respiratory function measuring apparatus according to claim 8, wherein
a waveform display is performed with plotting the respiratory signal as an abscissa, and a respiratory volume which is obtained by integrating a respiratory flow, as an ordinate.

## Patentansprüche

1. Atmungsfunktionsmessvorrichtung, die umfasst:
einen ersten Sensor (11), der konfiguriert ist, um einen invasiven Blutdruck zu erfassen;
einen zweiten Sensor (12, 13, 14), der konfiguriert ist, um die Frequenz von wenigstens einem Herzschlag oder einer Atmung zu messen; und
eine Steuerung (30), die konfiguriert ist, um ein Atmungsfunktionssignal von dem durch den ersten Sensor (11) erfassten invasiven Blutdruck zu extrahieren, indem wenigstens die von dem zweiten Sensor (12, 13, 14) gemessene Frequenz oder eine Oberwelle der Frequenz verwendet wird;
wobei die Steuerung konfiguriert ist, um Atmungsschwankungen eines intrathorakalen Drucks mittels des extrahierten Atmungsfunktionssignals zu schätzen,
**dadurch gekennzeichnet, dass**
das Schätzen der Atmungsschwankungen das Erfassen einer Position des extrahierten Atmungsfunktionssignals mit einem höchsten Wert in einer Ausatmungsphase oder das Erfassen eines Endes des Atemzugs, die Einstellung des höchsten Wertes bzw. des Endes des Atemzugs als einen Referenzpunkt, das Messen der Änderung des extrahierten Atmungsfunktionssignals mit Bezug auf den Referenzpunkt und das Einstellen der gemessenen Änderung als Atmungsschwankung des intrathorakalen Drucks umfasst; und
wobei die Atmungsfunktionsmessvorrichtung konfiguriert ist, um das extrahierte Atmungsfunktionssignal und die geschätzten Atmungsschwankungen des intrathorakalen Drucks anzuzeigen.

2. Atmungsfunktionsmessvorrichtung nach Anspruch 1, wobei,
wenn der zweite Sensor konfiguriert ist, um die Frequenz des Herzschlags zu messen, die Steuerung konfiguriert ist, um Komponenten entsprechend der Frequenz des Herzschlags und einer Oberwelle der Frequenz aus einem Signal entsprechend dem invasiven Blutdruck selektiv zu entfernen.

3. Atmungsfunktionsmessvorrichtung nach Anspruch 1, wobei,
wenn der zweite Sensor konfiguriert ist, um die Frequenz des Herzschlags zu messen, die Steuerung konfiguriert ist, um eine Komponente entsprechend einer Frequenz, die niedriger ist als die Frequenz des Herzschlags, aus einem Signal entsprechend dem invasiven Blutdruck zu extrahieren.

4. Atmungsfunktionsmessvorrichtung nach Anspruch 1, wobei,
wenn der zweite Sensor konfiguriert ist, um die Frequenz der Atmung zu messen, die Steuerung konfiguriert ist, um Komponenten entsprechend der Frequenz der Atmung und einer Oberwelle der Frequenz aus einem Signal entsprechend dem invasiven Blutdruck zu extrahieren.

5. Atmungsfunktionsmessvorrichtung nach Anspruch 1, wobei
die Frequenz des Herzschlags anhand eines Elektrokardiogramms, eines Plethysmogramms oder eines arteriellen Blutdrucks gemessen wird.

6. Atmungsfunktionsmessvorrichtung nach Anspruch 1, wobei
die Frequenz der Atmung anhand einer Kapnometrie, eines Atemflusses, eines Atemwegsdrucks, einer transthorakalen elektrischen Impedanz oder einer Atmungstemperatur gemessen wird.

7. Atmungsfunktionsmessvorrichtung nach Anspruch 1, wobei
der invasive Blutdruck ein zentraler Venendruck (Central Venous Prossure - CVP) oder ein peripherer Venendruck ist.

8. Atmungsfunktionsmessvorrichtung nach Anspruch 1, die des Weiteren umfasst:
einen Atmungsschwankungsdetektor, wobei,
wenn der zweite Sensor konfiguriert ist, um die Frequenz der Atmung zu messen, der zweite Sensor konfiguriert ist, um ein Ende des Atemzugs zu erfassen, und
der Atmungsschwankungsdetektor konfiguriert ist, um eine Änderung des Atmungsfunktionssignals mit Bezug auf das Ende des Atemzugs zu messen.

9. Atmungsfunktionsmessvorrichtung nach Anspruch 8, wobei
das Ende des Atemzugs anhand eines flachen Abschnitts des invasiven Blutdrucks, einer Kapnometrie, eines Atemflusses, eines Atemwegsdrucks, einer transthorakalen elektrischen Impedanz oder einer Atmungstemperatur gewonnen wird.

10. Atmungsfunktionsmessvorrichtung nach Anspruch 8, die des Weiteren umfasst:
eine Atmungsbestimmungsvorrichtung, die konfiguriert ist, um auf der Basis einer Polarität eines Grades des Atmungsfunktionssignals zu bestimmen, ob die Atmung eine spontane Atmung oder eine künstliche Atmung ist.

11. Atmungsfunktionsmessvorrichtung nach Anspruch 8, die des Weiteren umfasst:
einen sekundären Atmungsfunktionsrechner, der konfiguriert ist, um auf der Basis eines Pegels des Atmungsfunktionssignals wenigstens einen PTP, einem intrinsischen PEEP, eine Atmungsarbeit oder einen P0.1 zu erhalten.

12. Atmungsfunktionsmessvorrichtung nach Anspruch 8, wobei
das Atmungsfunktionssignal als ein ausgestrichener Teil auf der Basis eines Zeitpunktes des Endes des Atemzugs angezeigt wird.

13. Atmungsfunktionsmessvorrichtung nach Anspruch 8, wobei
eine Markierung, die einen Zeitpunkt zum Ende des Atemzugs anzeigt, mit einer Wellenform angezeigt wird.

14. Atmungsfunktionsmessvorrichtung nach Anspruch 8, wobei
eine Wellenformanzeige mit dem Einzeichnen des Atmungssignals als eine Abszisse und eines Atmungsvolumen, das durch Integrieren eines Atemflusses gewonnen wird, als eine Ordinate durchgeführt wird.

## Revendications

1. Appareil de mesure de la fonction respiratoire comprenant :
un premier capteur (11) configuré pour détecter une pression sanguine invasive ;
un second capteur (12, 13, 14) configuré pour mesurer la fréquence d'au moins l'un d'un battement du coeur et d'une respiration ; et
un dispositif de commande (30) configuré pour extraire un signal de fonction respiratoire de la pression sanguine invasive détectée par le premier capteur (11), en utilisant au moins l'une de la fréquence mesurée par le second capteur (12, 13, 14) et d'une harmonique de la fréquence ;
le dispositif de commande étant configuré pour estimer les variations respiratoires d'une pression intrathoracique en utilisant le signal de fonction respiratoire extrait,
**caractérisé en ce que** l'estimation des variations respiratoires comprend la détection d'une position du signal de fonction respiratoire extrait ayant une valeur la plus élevée dans une phase d'expiration ou la détection d'une fin d'expiration, paramétrant la valeur la plus élevée ou la fin d'expiration, respectivement, comme point de référence, la mesure du changement du signal de fonction respiratoire extrait par rapport au point de référence et le paramétrage du changement mesuré comme variation respiratoire de la pression intrathoracique ; et
l'appareil de mesure de la fonction respiratoire étant configuré pour afficher le signal de fonction respiratoire extrait et les variations respiratoires estimées de la pression intrathoracique.

2. Appareil de mesure de la fonction respiratoire selon la revendication 1, dans lequel
lorsque le second capteur est configuré pour mesurer la fréquence du battement du coeur, le dispositif de commande est configuré pour retirer sélectivement les composants correspondant à la fréquence du battement du coeur et une harmonique de la fréquence à partir d'un signal correspondant à la pression sanguine invasive.

3. Appareil de mesure de la fonction respiratoire selon la revendication 1, dans lequel
lorsque le second capteur est configuré pour mesurer la fréquence du battement du coeur, le dispositif de commande est configuré pour extraire un composant correspondant à une fréquence qui est inférieure à la fréquence du battement du coeur à partir d'un signal correspondant à la pression sanguine invasive.

4. Appareil de mesure de la fonction respiratoire selon la revendication 1, dans lequel
lorsque le second capteur est configuré pour mesurer la fréquence de la respiration, le dispositif de commande est configuré pour extraire les composants correspondant à la fréquence de la respiration et une harmonique de la fréquence à partir d'un signal correspondant à la pression sanguine invasive.

5. Appareil de mesure de la fonction respiratoire selon la revendication 1, dans lequel
la fréquence du battement du coeur est mesurée à partir d'un électrocardiogramme, d'un pléthysmogramme, ou d'une pression sanguine artérielle.

6. Appareil de mesure de la fonction respiratoire selon la revendication 1, dans lequel
la fréquence de la respiration est mesurée par capnométrie, flux respiratoire, pression des voies aériennes supérieures, impédance électrique transthoracique, ou température respiratoire.

7. Appareil de mesure de la fonction respiratoire selon la revendication 1,
la pression sanguine invasive étant une pression veineuse centrale (CVP) ou une pression veineuse périphérique.

8. Appareil de mesure de la fonction respiratoire selon la revendication 1, comprenant en outre :
un détecteur de variation de respiration, dans lequel
lorsque le second capteur est configuré pour mesurer la fréquence de la respiration, le second capteur est configuré pour détecter la fin d'expiration, et
le détecteur de variation de respiration est configuré pour mesurer un changement du signal de fonction respiratoire par rapport à la fin d'expiration.

9. Appareil de mesure de la fonction respiratoire selon la revendication 8,
la fin d'expiration étant obtenue à partir d'une partie plate de la pression sanguine invasive, d'une capnométrie, d'un flux respiratoire, d'une pression des voies aériennes supérieures, d'une impédance électrique transthoracique, ou d'une température respiratoire.

10. Appareil de mesure de la fonction respiratoire selon la revendication 8, comprenant en outre :
un dispositif de détermination de respiration configuré pour, sur la base d'une polarité d'un degré du signal de fonction respiratoire, déterminer si la respiration est une respiration spontanée ou une respiration artificielle.

11. Appareil de mesure de la fonction respiratoire selon la revendication 8, comprenant en outre :
un calculateur de fonction respiratoire secondaire configuré pour, sur la base d'un niveau du signal de fonction respiratoire, obtenir au moins l'un d'un PTP, d'un PEEP intrinsèque, d'un travail de respiration, et d'un P0.1.

12. Appareil de mesure de la fonction respiratoire selon la revendication 8, dans lequel
le signal de fonction respiratoire est affiché sous la forme d'une partie représentée sur la base d'un chronométrage de la fin d'expiration.

13. Appareil de mesure de la fonction respiratoire selon la revendication 8, dans lequel
une marque indicatrice d'un chronométrage de la fin d'expiration est affichée avec une forme d'onde.

14. Appareil de mesure de la fonction respiratoire selon la revendication 8, dans lequel
l'affichage d'une forme d'onde est exécuté par le tracé du signal respiratoire en abscisses, et d'un volume respiratoire qui est obtenu par intégration d'un flux respiratoire, en ordonnées.
